# EUROPÄISCHE PATENTSCHRIFT

(11) **EP 2 976 311 B1**
(45) Veröffentlichungstag und Bekanntmachung des Hinweises auf die Patenterteilung: **23.12.2020**
(21) Anmeldenummer: 14705318.5
(22) Anmeldetag: 12.02.2014
(51) Int. Cl.: C04B 35/22, C04B 35/447, C04B 35/468, C04B 35/626, C04B 35/653, A61L 27/10, A61L 27/12, C09K 3/14, C03C 3/112, C03C 10/00, C03C 10/16, A61L 31/02, B24C 1/06, B24C 1/08, B24C 11/00, C03C 4/00, B82Y 30/00

(54) **SINTER- UND/ODER SCHMELZFÄHIGE KERAMISCHE MASSE, DEREN HERSTELLUNG UND VERWENDUNG**
SINTERABLE AND/OR FUSIBLE CERAMIC MASS, PRODUCTION AND USE THEREOF
MATÉRIAU CÉRAMIQUE FRITTABLE ET/OU FUSIBLE, SA FABRICATION ET SON UTILISATION

(30) Priorität: 21.03.2013 DE 102013102917
(43) Veröffentlichungstag der Anmeldung: 27.01.2016
(73) Patentinhaber: Bundesrepublik Deutschland, vertreten durch das Bundesministerium für Wirtschaft und Technologie, 12205 Berlin (DE)
(72) Erfinder: BERGER, Georg, 16341 Panketal (DE); SPITZER, Andrea, 10827 Berlin (DE); NICOLAIDES, Dagmar, 15831 Mahlow (DE); GÜNSTER, Jens, 14169 Berlin (DE); MARX, Heidi, 10315 Berlin (DE)
(74) Vertreter: Zimmermann & Partner Patentanwälte mbB
(86) Internationale Anmeldenummer: PCT/EP2014/052733
(87) Internationale Veröffentlichungsnummer: WO 2014/146831

(56) Entgegenhaltungen:
- JP-A- S61 158 841
- KR-B1- 920 000 150
- US-A- 4 652 534
- US-A1- 2004 023 784
- ORTEGA-LARA W ET AL: "In vitro bioactivity of wollastonite-titania materials obtained by sol-gel method or solid state reaction", JOURNAL OF SOL-GEL SCIENCE AND TECHNOLOGY, KLUWER ACADEMIC PUBLISHERS, BO, Bd. 48, Nr. 3, 13. August 2008 (2008-08-13), Seiten 362-368, XP019646219, ISSN: 1573-4846, DOI: 10.1007/S10971-008-1817-X
- DEEPAK K. PATTANAYAK ET AL: "Apatite Wollastonite-Titanium Biocomposites: Synthesis and In Vitro Evaluation", JOURNAL OF THE AMERICAN CERAMIC SOCIETY, Bd. 0, Nr. 0, 1. Juni 2006 (2006-06-01), XP055116128, ISSN: 0002-7820, DOI: 10.1111/j.1551-2916.2006.01068.x
- CHENGTIE WU ET AL: "Incorporation of titanium into calcium silicate improved their chemical stability and biological properties", JOURNAL OF BIOMEDICAL MATERIALS RESEARCH PART A, Bd. 86A, Nr. 2, 1. August 2008 (2008-08-01), Seiten 402-410, XP055116169, ISSN: 1549-3296, DOI: 10.1002/jbm.a.31623

## Beschreibung

Die Erfindung liegt auf dem Gebiet von sinter- und/oder schmelzfähigen keramischen Massen, nachfolgend keramische Masse genannt, und Formkörpern umfassend keramische Massen, insbesondere auf dem Gebiet von bioaktiven bzw. bioreaktiven, d.h. einen direkten und bindegewebsfreien Knochenkontakt bewirkenden keramischen Massen, langzeitstabilem Knochenersatz sowie Herstellungs- und Strukturierungsverfahren dafür.

Unter einer keramischen Masse wird in diesem Zusammenhang ein anorganischer nichtmetallischer Werkstoff verstanden, sowohl in gesinterter, gebrannter Form, als auch in Form eines zum Sintern oder Brennen vorbereiteten Materials. Ein keramischer Formkörper wird in der Regel bei Raumtemperatur aus einer Rohmasse, beispielsweise einem Schlicker oder einer Paste geformt und durch einen Sintervorgang bei hohen Temperaturen verfestigt. Die Vorbereitung eines Materials zum Sintern kann auch ein erstes Sintern und ein nachfolgendes Zermahlen des gesinterten Materials umfassen, beispielsweise indem das zermahlene Material anschließend wieder zur Herstellung eines Schlickers oder einer Paste verwendet wird, aus dem ein Grünkörper geformt wird. Beispielsweise kann der Grünkörper eine Sinterglaskeramik ergeben. Diese Vorbereitung kann aber ebenso das Zermahlen eines zuvor geschmolzenen Glases umfassen. Ferner ist es möglich, dass das geschmolzene Glas einer Temperaturbehandlung unterzogen wird, so dass - dem klassischen glaskeramischen Verfahren entsprechend - eine Kristallisation innerhalb der Glasmatrix erfolgt.

Die keramische Masse kann entweder als Granulat vorliegen, das nach geeigneter Vorbehandlung direkt zum Ersatz natürlichen Knochenmaterials oder der Ergänzung eines Knochens in einem lebenden Organismus verwendet wird. Ebenso aber kann die keramische Masse zu einem Formkörper verarbeitet werden, der nach geeigneter Vorbehandlung einen Knochen in einem lebenden Organismus vollständig ersetzt. Einsetzbare Verarbeitungsverfahren umfassen zusatzlich auch additive Fertigungsverfahren, z.B. das sogenannte 3D-Printing und andere Rapid-Prototyping Verfahren und optional daran anschließende gesonderte Sinterschritte oder auch die Imprägnierung oder Besiedlung dabei erhaltener Strukturen mit lebenden Zellen.

Bekannte langzeitstabile und bioaktive/bioreaktive Glaskeramiken bzw. keramische Massen bestehen überwiegend aus Apatit und Wollastonit. Sie können als Sinterglaskeramik (Kokubo [1]) oder nach klassischen glaskeramischen Verfahren (Berger et al. [2]) hergestellt werden. Reine Hydroxylapatit-Werkstoffe, die a priori als bioaktiv und langzeitstabil gelten, können in der Regel nicht als beliebige, speziell gedruckte Formkörper hergestellt werden und weisen in der Regel für den Einsatz als Knochenersatzmaterial unzureichende Festigkeitswerte auf. Dabei wird in diesem Zusammenhang unter einem langzeitstabilen Knochenersatz, bzw. einem langzeitstabilen Verbund im Gegensatz zu einem resorbierbaren Material oder einem resorbierbaren Materialverbund ein solches Material verstanden, das während des Anwendungszeitraums, welcher beispielsweise der typischen Lebensdauer des jeweiligen Organismus (Wirbeltier, Mensch) entspricht, nicht resorbiert und nicht durch natürlichen Knochen ersetzt wird, sondern im Wesentlichen erhalten bleibt. Seine Löslichkeit im lebenden Organismus bzw. unter physiologischen Bedingungen einer Zell- und/oder Gewebekultur ist somit äußerst gering. Das äußert sich darin, dass über den gesamten Anwendungszeitraum hinweg eine Grenzfläche des Materials erhalten bleibt, an die sich im fortlaufenden Remodeling-Prozess im Organismus natürliche Knochensubstanz anlagern kann, bzw. die bei einer technischen Anwendung abrasiv wirkt.

Insbesondere ist die Löslichkeit bekannter Produkte auf der Basis von Glaskeramiken, die ausschließlich die Hauptkristallphasen Apatit und Wollastonit aufweisen, noch zu groß. Wird die Löslichkeit durch Zusätze an Al₂O₃, ZrO₂, oder TiO₂ erniedrigt, so erhöht sich zwar in Abhängigkeit von der Menge des Zusatzes die Beständigkeit des Materials unter physiologischen Bedingungen bzw. in physiologischer Lösung, jedoch wird dabei die bindegewebsfreie direkte Knochenkontaktrate verringert, da sich diese Stoffe (Al₂O₃, ZrO₂, TiO₂) vorlagern und eigenständige Schichten bilden [3].

KR 920 000 150 B1 lehrt ein kristallisiertes Glas für den menschlichen Körper und ein Herstellungsverfahren dafür. US 4 652 534 A lehrt eine hochfeste Glaskeramik mit einer Biegefestigkeit von zumindest 1700 kg/cm², umfassend kristallines Apatit und zumindest 50 Gew.-% kristallines Wollastonit. JP S61 158841 A lehrt ein Glaspulver < 200 mesh, aus dem durch Wärmebehandlung Apatit und Diopsid-Kristalle und/oder Wollastonit-Kristalle ausgeschieden werden können. W. Ortega-Lara et al. (2008) offenbaren im Artikel "In vitro bioactivity of wollastonite-titania materials obtained by sol-gel method or solid state reaction" im Journal of Sol-Gel Science and Technology 48:362-368 den Erhalt eines Knochenersatzmaterials. Deepak K. Pattanayak et al. (2006) beschreiben im Artikel "Apatite Wollastonite-Titanium Biocomposites: Synthesis and in-vitro Evaluation" im Journal of the American Ceramic Society 89(7):2172-2176 die Ausbildung eines glaskeramischen Apatit-Wollastonit-Titan-Kompositmaterials mit verschiedenenen Titan-Anteilen (5-40 Gew.-%) in einer Argon-Atmosphäre, dessen Sintereigenschaften und Verhalten in simulierter Körperflüssigkeit. Chengtie Wu et al. (2008) beschreiben im Journal of Biomedical Materials Research, 86A(2):402-410 unter dem Titel "Incorporation of titanium into calcium silicate improved their chemical stability and biological properties" ein bioaktives Material, das neben Calciumsilikat Titan umfasst. G. Berger und V. Atzrodt (1984) beschreiben in physica status solidi (a) 85(1): K9-K13 im Artikel "In vitro characterization of bioactivity of glassy or glasscrystalline implant materials using AUGER electron spectroscopy" die Herausbildung einer die Korrosion in wässrigen Lösungen verringernden Schutzschicht auf Glaskeramiken durch Zusatz von Al₂O₃ oder ZrO₂.

Vor diesem Hintergrund wird eine keramische Masse nach Anspruch 1, ein Herstellungsverfahren für diese keramische Masse nach Anspruch 4, Verwendungen der keramischen Masse bzw. daraus erhaltener Formkörper nach den Ansprüchen 12 bis 19 und ein Formkörper nach Anspruch 20 vorgeschlagen. Weitere Ausführungsformen, Modifikationen und Verbesserungen ergeben sich anhand der folgenden Beschreibung und der beigefügten Ansprüche.

Gemäß einer ersten Ausführungsform wird eine keramische Masse nach Anspruch 1 vorgeschlagen. Diese umfasst einen langzeitstabilen Verbund kristalliner Phasen von Apatit, Wollastonit, Cristobalit und Titanit, der durch eine Glasphase stabilisiert ist, wobei unter dem langzeitstabilen Verbund im Gegensatz zu einem resorbierbaren Material ein solches Material verstanden wird, das während eines Anwendungszeitraums, welcher einer typischen Lebensdauer eines Menschen entspricht, nicht resorbiert wird und nicht durch natürlichen Knochen ersetzt wird, wobei zur Herstellung der keramischen Masse verwendete Ausgangsstoffe 35-45 Masse-% SiO₂, 25-35 Masse-% CaO, 10-15 Masse-% P₂O₅, 2-4 Masse-% MgO, 4-6 Masse-% CaF₂ und 5-10 Masse-% TiO₂ allein oder in Kombination mit zumindest einem Alkalioxid, ausgewählt unter 0,001-5 Masse-% Na₂O und 0,001-0,2 Masse-% K₂O umfassen, wobei ein Anteil der Glasphase in der keramischen Masse nach einem Sintern weniger als 25 Masse-% beträgt, und wobei die keramische Masse herstellbar ist durch ein erstes Sintern drucklos bei einer Temperatur im Bereich von 650 °C bis 800 °C über eine Dauer von 1 bis 48 Stunden, ein Zermahlen der beim ersten Sintern gesinterten keramischen Masse, und ein zweites Sintern drucklos bei einer Temperatur im Bereich von 850 °C bis 1200 °C über eine Dauer von 1 bis 48 Stunden. Insbesondere zeichnet sich die keramische Masse dadurch aus, sinter- und/oder schmelzfähig zu sein.

Ein Vorteil dieser Ausführungsform ergibt sich aus der stabilen Einbettung der bezeichneten kristallinen Phasen in eine gemeinsame Glasphase, die in physiologischen Medien zumindest teilweise löslich ist und somit vorteilhafte Verankerungsmöglichkeiten für einwachsende Zellen und die Anlagerung von natürlicher Knochensubstanz bietet, ohne dass die keramische Masse sich beispielsweise im Körper auflöst oder resorbiert wird. Daraus resultiert vorteilhaft eine ausgezeichnete Osseointegration bzw. eine hohe Knochenkontaktrate.

Gemäß der ersten Ausführungsform umfasst die keramische Masse neben den genannten Hauptkristallphasen Apatit, Wollastonit und Titanit weiterhin Cristobalit.

Ein spezieller Vorteil ergibt sich aus der stabilen Einbettung aller bezeichneten kristallinen Phasen in eine gemeinsame Glasphase, einer ausgeprägten Stabilität der keramischen Masse in physiologischen Medien und einer ausgezeichnete Osseointegration bzw. hohen Knochenkontaktrate.

Gemäß der genannten ersten Ausführungsform umfassen die zur Herstellung der keramischen Masse verwendeten Ausgangsstoffe SiO₂, CaO, P₂O₅, MgO, CaF₂ und TiO₂ allein oder in Kombination mit zumindest einem Alkalioxid, ausgewählt unter Na₂O und K₂O.

Vorteilhaft ist, dass die Löslichkeit der Glasphase durch die stöchiometrischen Verhältnisse der genannten Stoffe eingestellt und somit eine vorteilhaft hohe Knochenkontaktrate erreicht wird.

In diesem Zusammenhang werden unter stöchiometrischen Bestandteilen die Stoffmengen- bzw. Masseverhältnisse der genannten Stoffe verstanden, wobei die stöchiometrischen Verhältnisse der genannten Oxide bzw. des Kalziumfluorid durch Einwaage unterschiedlicher Ausgangsstoffe erzielt werden können. Beispielsweise könnte für den Eintrag einer gewünschten Stoffmenge Kalzium, Kalium bzw. Natrium auch das entsprechende Salz der Oxalsäure eingewogen werden.

Gemäß der ersten Ausführungsform beträgt der Anteil der Glasphase in der keramischen Masse nach einem Sintern wenig er als 25 Masse-%, gemäß einer dritten Ausführungsform weniger als 10 Masse-%.

Ein wesentlicher Vorteil des wie bezeichnet geringen Anteils der Glasphase besteht in der erhöhten Stabilität der gesinterten keramischen Masse in physiologischen Medien, insbesondere in Gegenwart von Serum und bei Besiedlung mit lebenden Zellen, insbesondere während der Osseointegration.

Gemäß einer zweiten Ausführungsform erfolgt das erste Sintern bei 720 °C über eine Dauer von 8 bis 24 Stunden und das zweite Sintern bei 920 bis 1150 °C über eine Dauer von 18 bis 24 Stunden.

Gemäß einer vierten Ausführungsform wird ein Herstellungsverfahren für eine sinter- und/oder schmelzfähige keramische Masse vorgeschlagen, das die nachfolgenden Schritte umfasst:
- Bereitstellen einer Mischung, umfassend zumindest die Bestandteile 35-45 Masse-% SiO₂, 25-35 Masse-% CaO, 10-15 Masse-% P₂O₅, 2-4 Masse-% MgO, 4-6 Masse-% CaF₂ und 5-10 Masse-% TiO₂ allein oder in Kombination mit zumindest einem Alkalioxid, ausgewählt unter 0,001-5 Masse-% Na₂O und 0,001-0,2 Masse-% K₂O; wobei ein Masse-Verhältnis der Summe der Bestandteile SiO₂, CaO und P₂O₅ in der Mischung zur Masse des in der Mischung enthaltenen TiO₂ von 7:1 bis 20:1 beträgt;
- Vermahlen der Mischung;
- Sintern bzw. Tempern der vermahlenen Mischung in einem ersten Temperaturbehandlungsschritt zu einer ersten Masse, wobei das Sintern bzw. Tempern bei einer Temperatur im Bereich von 650 °C bis von 800 °C, insbesondere bei 720 °C, über eine Dauer von mindestens 1 Stunde, bevorzugt über eine Dauer von 8 bis 24 Stunden erfolgt;
- Zermahlen der ersten Masse;
- Aufschlämmen zu einem Schlicker der zermahlenen ersten Masse;
- Formen eines Grünkörpers aus dem Schlicker;
- Sintern des Grünkörpers in einem zweiten Temperaturbehandlungsschritt, wobei das Sintern des Grünkörpers bei einer Temperatur im Bereich von 850°C bis 1200°C, insbesondere bei 920°C bis 1150°C, über eine Dauer von mindestens 18 Stunden bis 24 Stunden erfolgt,
   wobei unter dem langzeitstabilen Verbund im Gegensatz zu einem resorbierbaren Material ein solches Material verstanden wird, das während eines Anwendungszeitraums, welcher einer typischen Lebensdauer eines Menschen entspricht, nicht resorbiert wird und nicht durch natürlichen Knochen ersetzt wird und
   wobei ein Anteil der Glasphase in der keramischen Masse weniger als 25 Masse-% beträgt.

Vorteile dieses Herstellungsverfahrens bestehen in der Möglichkeit, eine sinter- und/oder schmelzfähige keramische Masse bereitzustellen, die angepasst ist für die Herstellung von Knochenersatzmaterial und künstlichen Knochen. Ein besonderer Vorteil des Herstellungsverfahrens besteht in der Bereitstellung von rieselfähigen Pulvern und Granulaten die sich besonders für additive Fertigungsverfahren eignen.

Gemäß einer weiteren Ausführungsform geht dem Sintern bzw. Tempern im ersten Temperaturbehandlungsschritt ein Schmelzen der vermahlenen Mischung bei einer Schmelztemperatur unterhalb von 1650 °C, insbesondere unterhalb von 1620 °C voraus. Nach einer beispielhaften Ausführungsform kann das Schmelzen beispielsweise in einem Tiegel, der z. B. aus Platin/Rhodium besteht, vorgenommen werden.

Das eröffnet besondere Vorteile für den Erhalt eines feinkristallinen Gefüges von Apatit, Wollastonit, Titanit und Cristobalit, eingebettet in eine Glasmatrix bzw. einer Glaskeramik, umfassend ein feinkristallines Gefüge von Apatit, Wollastonit, Titanit und Cristobalit.

Gemäß der genannten ersten Ausführungsform umfasst die bereitgestellte Mischung 35-45 Masse-% SiO₂; 25-35 Masse-% CaO, 10-15 Masse-% P₂O₅; 2-4 Masse-% MgO, 4-6 Masse-% CaF₂ und 5-10 Masse-% TiO₂ und kann 0,001-5 Masse-% Na₂O und/oder 0,001-0,2 Masse-% K₂O enthalten.

Vorteile dieser Ausführungsformen ergeben sich aus der bei dieser hier bezeichneten Zusammensetzung erfolgenden synchronen Ausscheidung der Kristallphasen Apatit, Wollastonit, Titanit und optional Cristobalit, ohne das nach Abschluss des Sinterprozesses vorgelagerte titanoxidreiche Schichten auftreten, die beispielsweise einen direkten Knochenkontakt im Rezipienten verhindern.

Gemäß einer weiteren Ausführungsform des Herstellungsverfahrens beträgt ein mittlerer Durchmesser der Partikel zum Erhalt der keramischen Masse gemischten Bestandteile 0,5 - 3000 µm, bevorzugt 0,5 - 3 µm; insbesondere 2 - 10 µm; 10 - 100 µm; 50 - 1000 µm oder 300 - 3000 µm.

Vorteile dieser Ausführungsform bestehen im gleichmäßigen Auftreten bzw. der homogenen Verteilung der ausgeschiedenen Kristallphasen Apatit, Wollastonit, Titanit und typischerweise zusätzlich Cristobalit. Die gleichmäßige Verteilung dieser Kristallite hat eine gute Festigkeit der erhaltenen Formkörper unabhängig von einer Hauptbelastungsrichtung zur Folge.

Gemäß einer weiteren Ausführungsform umfasst das vorgeschlagene Herstellungsverfahren das Befüllen einer Sinterform. Die Sinterform ist so an die Größe und Gestalt des letztendlich benötigten Formkörpers angepasst, dass nach dem Sintern keine weitere Bearbeitung mehr erforderlich ist. Das wird insbesondere dadurch ermöglicht, dass mit der vorgeschlagenen Zusammensetzung der Ausgangsstoffe keine vorgelagerten Schichten, beispielsweise titanoxidreichen Schichten, mehr abgetragen werden müssen, da solche nicht auftreten. Der gesinterte Formkörper kann deshalb unmittelbar verwendet werden, beispielsweise als Ersatzknochen.

Gemäß der genannten ersten Ausführungsform des vorgeschlagenen Herstellungsverfahrens einer sinter- und/oder schmelzfähigen keramischen Masse erfolgt das Sintern in zwei Schritten drucklos.

Vorteile dieser Ausführungsform ergeben sich aus einer wesentlich vereinfachten Verfahrensführung. Beispielsweise kann ein Sinterschritt das Sintern eines Pulvers als Pulver-Sprüh-Sintern umfassen.

Gemäß der genannten ersten Ausführungsform erfolgt das Sintern drucklos in einem ersten Schritt bei einer Temperatur von 650 bis 800 °C, insbesondere bei 720 °C, während einer Zeitdauer von 1 bis 48 Stunden, insbesondere während einer Zeitdauer von 24 Stunden, und in einem zweiten Schritt drucklos bei einer Temperatur von 850 bis 1200 °C, insbesondere bei 920 bis 1150 °C während einer Zeitdauer von 1 bis 48 Stunden, insbesondere während einer Zeitdauer von 24 Stunden.

Vorteile dieser Ausführungsform bestehen darin in, dass die in Hinsicht auf die bevorzugte Knochenkontaktrate und die bevorzugte mechanische Belastbarkeit der erhaltenen Materialien vorteilhafte Phasenzusammensetzung bei vergleichsweise geringem technologischen Aufwand erreicht wird.

Gemäß der genannten ersten Ausführungsform geht dem zweiten Sinterschritt ein Vermahlen der nach dem ersten Sinterschritt erhaltenen keramischen Masse voraus.

Vorteile ergeben sich insbesondere aus einer homogenen Struktur hinsichtlich Porenverteilung und der gleichmäßigen Verteilung der unterschiedlichen kristallinen Phasen, sowie einer daraus resultierenden isotropen Festigkeit der erhaltenen Formkörper. Das bietet besondere Vorteile für die Eignung der erhaltenen Formkörper für die ihnen jeweils zugedachte Verwendung.

Gemäß einer weiteren Ausführungsform wird vorgeschlagen, dass ein Masse-Verhältnis der Summe der Ausgangsstoffe SiO₂, CaO, P₂O₅, MgO und CaF₂ zur Masse des eingesetzten TiO₂ eingestellt wird auf ein Verhältnis von 8:1 bis 20:1. Details zu vorteilhaften Ausführungsbeispielen finden sich in Tabelle 5.

Gemäß der vierten Ausführungsform wurde oben ein Herstellungsverfahren vorgeschlagen für eine sinter- und/oder schmelzfähige keramische Masse, die einen durch eine Glasphase stabilisierten langzeitstabilen Verbund kristalliner Phasen von Apatit, Wollastonit, Titanit und optional auch Cristobalit umfasst. Dieses Herstellungsverfahren zeichnet sich dadurch aus, dass ein Masse-Verhältnis der Summe von SiO₂, CaO und P₂O₅ in der Mischung der Ausgangsstoffe der keramischen Masse zu dem in der Mischung der Ausgangsstoffe enthaltenen TiO₂ von 7:1 bis 20:1 beträgt. Vorteilhafterweise kann bei diesem Massenverhältnis die Ausbildung einer vorgelagerten titanoxidreichen Phase vermieden werden.

Gemäß einer weiteren Ausführungsform des Herstellungsverfahrens erfolgt das Formen des Grünkörpers schichtweise oder unter Verwendung eines additiven Verfahrens unter Verwendung eines Schlickers der gemahlenen Ausgangsstoffe bzw. eines Schlickers aus der vermahlenen, nach einem ersten Sinterschritt erhaltenen keramischen Masse.

Additive Verfahren erfordern typischerweise eine dem jeweiligen Verfahren angepasste Verteilung der im verwendeten Pulver, Granulat oder Schlicker vorliegenden mittleren Teilchengrößen. Auf Grund einer entweder über Mahlschritte weitestgehend frei einstellbaren Größenverteilung bzw. mittleren Teilchengrößen oder einer mittels angepasstem Sprühsintern einstellbaren mittleren Teilchengröße ergibt sich somit die Möglichkeit, die jeweilige Teilchengröße dem bevorzugten Additivverfahren anzupassen. Somit können die bekannten Vorteile von Additivverfahren genutzt werden. Beispielsweise kann ein Schlicker mittels eines geeigneten Druckverfahrens verarbeitet werden. Das ermöglicht beispielsweise den Einsatz der beschriebenen keramischen Masse zur Erzeugung von dreidimensionalen, schichtweise aufgebauten Formkörpern.

Gemäß einer weiteren Ausführungsform erfolgt das Zermahlen der ersten gesinterten Masse zuschlagsstofffrei. Vorteile ergeben sich durch einen verringerten Zeitaufwand und verringerte Kosten.

Gemäß einer weiteren Ausführungsform wird vorgeschlagen, die sinter- und/oder schmelzfähige keramische Masse als Strahlmittel für medizinische Implantate zu verwenden, wobei eine mittlere Korngröße des Strahlmittels im Bereich von 50 - 5000 µm, bevorzugt im Bereich zwischen 50 µm bis 1000 µm liegt.

Aus der vorteilhaften physiologischen Verträglichkeit und hohen Knochenkontaktrate können die Nachteile bisheriger Strahlmittel, beispielsweise von Korund, vermieden werden. Die gestrahlten Oberflächen müssen keiner aufwendigen Reinigung unterzogen werden, das ggf. in oberflächlichen Hinterschneidungen zurückbleibende Strahlgut kann sogar das Einwachsen einer behandelten Oberfläche verbessern.

Gemäß einer weiteren Ausführungsform wird vorgeschlagen, die zuvor beschriebene sinter- und/oder schmelzfähige keramische Masse in Form einer giessbaren Masse in einem Schlickerguss-Verfahren einzusetzen, wobei eine mittlere Korngröße der in der giessbaren Masse dispergierten Teilchen im Bereich von 0,5 µm bis 7 µm, bevorzugt im Bereich von 0,5 µm bis 3 µm liegt.

Vorteile ergeben sich aus der Eignung der keramischen Masse für additive Fertigungsverfahren.

Gemäß einer weiteren Ausführungsform wird vorgeschlagen, die zuvor beschriebene sinter- und/oder schmelzfähige keramische Masse zum Verfestigen, Reinigen, Aufrauhen oder Polieren von Oberflächen medizinischer Implantate zu verwenden, wobei die keramische Masse als Formkörper vorliegt, und eine mittlere Ausdehnung des Formkörpers in einer Raumrichtung im Bereich von 0,25 µm bis 1 cm, insbesondere im Bereich von 0,5 µm bis 500 µm liegt.

Vorteile ergeben sich aus der universellen Einsetzbarkeit der keramischen Masse, sei es nun als Schleifkörper, Feinstrahlmittel oder Poliermittel für metallische oder Polymeroberflächen oder für die Oberflächen von Formkörpern, umfassend ein Verbundmaterial.

Gemäß einer weiteren Ausführungsform wird vorgeschlagen, die zuvor beschriebene sinter- und/oder schmelzfähige keramische Masse und einen daraus gewonnenen Formkörper als Schleifkörper für ein medizinisches Implantat oder für eine Endoprothese zu verwenden.

Aus der vorteilhaften physiologischen Verträglichkeit und hohen Knochenkontaktrate können die Nachteile bisheriger Schleifkörper, beispielsweise von Korund, vermieden werden. Die mit dem Schleifkörper behandelten Oberflächen müssen keiner aufwendigen Reinigung mehr unterzogen werden, das ggf. in oberflächlichen Hinterschneidungen zurückbleibende Material kann sogar das Einwachsen einer behandelten Oberfläche verbessern.

Gemäß einer weiteren Ausführungsform wird vorgeschlagen, die zuvor beschriebene sinter- und/oder schmelzfähige keramische Masse zur Behandlung einer metallischen Oberfläche zu verwenden.

Vorteile ergeben sich sowohl aus der Festigkeit der keramischen Formkörper als auch aus der hervorragenden Bioverträglichkeit der keramischen Masse bzw. der daraus hergestellten Formkörper.

Gemäß einer weiteren Ausführungsform wird vorgeschlagen, die zuvor beschriebene sinter- und/oder schmelzfähige keramische Masse in einer gießbaren Masse für den Schlickerguss einzusetzen, wobei die keramische Masse diskrete Partikeln mit einem mittleren Durchmesser der einzelnen Partikel im Bereich 0,5 µm bis 7 µm, bevorzugt im Bereich von 0,5 bis 3 µm umfasst.

Vorteile ergeben sich daraus, dass auf Schlickerguss basierende Verfahren eine weitestgehend große Freiheit für die Gestaltung der Form und Größe des Grünkörpers erlauben.

Gemäß einer weiteren Ausführungsform wird vorgeschlagen, die zuvor beschriebene sinter- und/oder schmelzfähige keramische Masse zur Herstellung eines Pressgranulats zu verwenden, wobei das Pressgranulat eine mittlere Granulatpartikelgröße von 0,5 µm bis 500 µm aufweist.

Vorteilhafterweise eignet sich die beschriebene keramische Masse zur Herstellung von Granulatpartikeln über einen weiten einstellbaren Größenbereich. Somit können anwendungsspezifische Granulate bereitgestellt werden, die die hier beschriebenen vorteilhaften Eigenschaften der gesinterten keramischen Masse aufweisen.

Gemäß einer weiteren Ausführungsform wird vorgeschlagen, die zuvor beschriebene sinter- und/oder schmelzfähige keramische Masse zur Herstellung eines rieselfähigen Pulvers für ein additives Fertigungsverfahren zu verwenden, wobei das rieselfähige Pulver eine mittlere Pulverpartikelgröße von 10 µm bis 125 µm aufweist.

Rieselfähigen Pulvern kommt sowohl für Nass- als auch für Trockenbett-Techniken aus Gründen eines effektiven Materialeinsatzes und verfahrensökonomischen Gründen eine große Bedeutung zu. Die frei einstellbare mittele Partikelgröße des rieselfähigen Pulvers erlaubt eine anwendungsbezogene Prozessoptimierung.

Gemäß einer weiteren Ausführungsform wird vorgeschlagen, die zuvor beschriebene sinter- und/oder schmelzfähige keramische Masse in Form eines Implantatgranulates bereitzustellen und zum Ersatz oder zur Ergänzung natürlichen Knochenmaterials einzusetzen, wobei das Implantatgranulat eine mittlere Partikelgröße von 300 µm bis 3000 µm aufweist.

Vorteile ergeben sich aus der besonderen Eignung eines derartigen Implantatgranulats, beispielsweise für die Kieferchirurgie und -orthopädie.

Gemäß einer weiteren Ausführungsform wird vorgeschlagen, die zuvor beschriebene sinter- und/oder schmelzfähige keramische Masse als Strahlgut zur Oberflächenbehandlung einzusetzen, wobei die keramische Masse eine mittlere Partikelgröße von 50 µm bis 5000 µm, beispielsweise von 50 µm bis 2500 µm aufweist.

Wie beschrieben ist die mittlere Partikelgröße frei einstellbar. Damit kann die hier beschriebene sinter- und/oder schmelzfähige keramische Masse mit unterschiedlichen Technologien verarbeitet bzw. daraus hergestellte Formkörper in unterschiedlichen Bereichen, beispielsweise als Knochenersatzmaterial, zum Knochenaufbau, zur Realisierung von Gerüst- und Stützstrukturen, als bioverträgliches Füllmaterial und als künstlicher Knochen eingesetzt werden.

Gemäß einer weiteren Ausführungsform ein Formkörper vorgeschlagen, umfassend eine hier beschriebene sinter- und/oder schmelzfähige keramische Masse, wobei der Formkörper ausgewählt ist unter: einem Mikropartikel, einer Kugel, einem Granulat, einem Polyeder, einer Scheibe, einem Diskus, einem Ellipsoid, einem Stab, einem Rohr, einem Zylinder, einem Kegel, einem knochenförmigen Gebilde oder einem Teil oder einem Abschnitt zumindest eines der genannten Formkörper.

Die bezeichnete Vielfalt erhältlicher Formen deckt eine weitestgehend breite Palette bevorzugter Anwendungen ab. Bevorzugt sind insbesondere solche Anwendungen, die eine gute Bioverträglichkeit, eine gute Knochenkontaktrate, eine schnelle Osseointegration und/oder eine gute Zell- und Gewebeverträglichkeit, insbesondere für Säugerzellen und Gewebe von Säugetieren erfordern.

Aus Sicht möglicher Einsatzgebiete kommt eine Verwendung der beschriebenen Materialien und Formkörper im Bereich der Veterinärmedizin, der Humanmedizin, der medizinischen Forschung und dazu in Beziehung stehenden Bereichen der Oberflächenbehandlung in Betracht.

Die vorstehend beschriebenen Ausführungsformen können beliebig miteinander kombiniert werden.

Die beiliegenden Figuren veranschaulichen Ausführungsformen und dienen zusammen mit der Beschreibung der Erläuterung der Prinzipien der Erfindung.
Fig. 1 zeigt eine mittels Rasterelektronenmikroskopie erhaltene Aufnahme des temperaturbehandelten Materials.
Fig. 2 zeigt die Kristallphasen- und Glasanteile in Abhängigkeit von der Temperaturbehandlung aus Tab. 4 als Blockdiagramm.
Fig. 3 zeigt die stöchiometrischen Zusammensetzungen der AWT-Variationen aus Tab. 5 als Blockdiagramm.

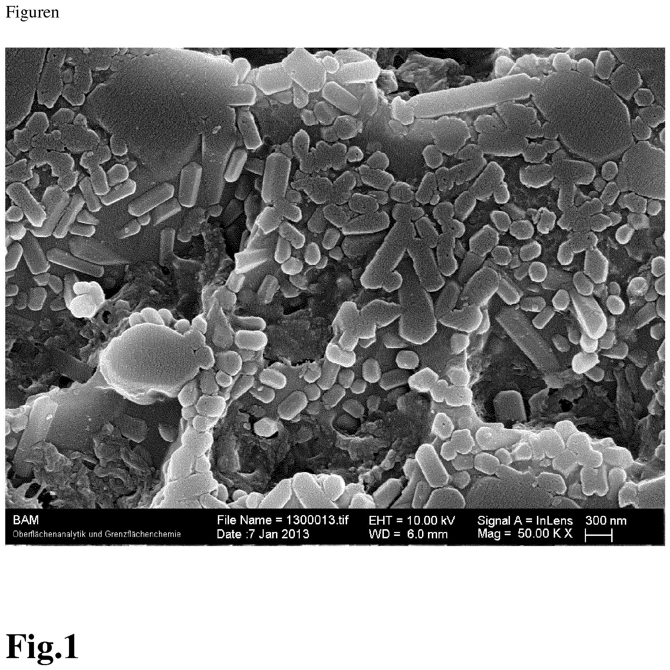

Insbesondere zeigt Figur 1 die feinkristalline Struktur des durch die vorgeschlagene Temperaturbehandlung erhaltenen Gefüges (vgl. Messbalken "300 nm" am Bildrand) der in eine Glasmatrix eingebetteten Kristalle. Diese Glaskeramik wurde aus der schmelzfähigen keramischen Masse AWT7-o.K-Si erhalten (vgl. Tab. 5, letzte Zeile).

Ausgehend von Zusammensetzungen, die zu einer keramischen Masse, insbesondere zu einer Glaskeramik, mit den Hauptkristallphasen Apatit und Wollastonit (AW) führen, wird durch eine bislang unüblich hohe Zugabe einer TiO₂-Komponente völlig überraschend eine dritte Hauptkristallphase erzeugt, die aus einem Titansilicat bzw. aus Titanit besteht. Typischerweise gelingt es, aus den vorgeschlagenen Zusammensetzungen eine vierte Hauptkristallphase, nämlich Cristobalit auszuscheiden, wenn nach dem Sintern und Erstarren der keramischen Masse ein weiterer Sinterschritt angeschlossen wird. Das Ausscheiden zweier zusätzlicher Hauptkristallphasen, d.h. das Auftreten der genannten dritten

(Titanit) und vierten Hauptkristallphasen (Cristobalit) bewirkt eine Verringerung des Anteils der Glasphase an der keramischen Masse. Es gelingt den Anteil der Glasphase in der vorgeschlagenen keramischen Masse im Vergleich zu einer Glaskeramik auf Basis von Apatit und Wollastonit auf lediglich etwa 10 Masse-% zu verringern. Dies trägt ebenfalls zur Erhöhung der chemischen Beständigkeit bzw. zur Langzeitstabilität der vorgeschlagenen keramischen Masse in physiologischer Lösung bzw. im lebenden Organismus bei.

Dabei wird unter einer keramischen Masse allgemein eine Zusammensetzung von Ausgangsstoffen, beispielsweise ein Pulvergemisch, verstanden. Die Zusammensetzung bzw. das Pulvergemisch wird nach gründlicher Durchmischung, die beispielsweise auch auf dem Wege des Mahlens und/oder Homogenisierens erreicht werden kann, zu einem Grünkörper verarbeitet, der im Ergebnis eines Sintervorganges einen Festkörper, bzw. eine feste keramische Masse mit allen hier beschriebenen Eigenschaften bildet. Die gesinterte keramische Masse umfasst zumindest die vier genannten Hauptkristallphasen und eine Glasphase.

Trotz der wie beschrieben erzielten Verminderung des Anteils der Glasphase an der keramischen Masse ist der Anteil der Glasphase an der vorgeschlagenen keramischen Masse jedoch immer noch ausreichend hoch, sodass aus granulierter, geschmolzener glaskeramischer Masse 3D-Formkörper gedruckt werden können, die nach drucklosem Sintern über eine ausreichende mechanische Stabilität verfügen (vgl. Tab.1), um beispielsweise als Knochenersatz verwendet werden zu können.

**Tab. 1 Festigkeiten von AWT7-o.K-Si 3D-Printing-Formkörper**

| Korngröße [µm] | Festigkeit [MPa] |
|---|---|
| 25-45 | 4,1 |
| 45-100 | 2,6 |

Dabei versteht es sich von selbst, dass aus der keramischen Masse bzw. der glaskeramischen Masse hergestellte Formkörper nach traditioneller keramischer Verarbeitung mit Druck bei der Formkörperherstellung eine höhere mechanische Festigkeit aufweisen, als die zuvor erwähnten drucklos hergestellten Formkörper. Das belegen beispielsweise die als Ausführungsbeispiel in Form von Tabletten gepressten Formkörper (vgl. Tab.2).

**Tab. 2 Festigkeiten von AWT7-o.K-Si gepresste Formkörper**

| Korngröße [µm] | Festigkeit [MPa] |
|---|---|
| 0,5-3,0 | 38,4 |
| 1,5-35,0 | 120,7 |

Dem Fachmann ist bekannt, dass beim Zusatz hoher Anteile Titanoxids zu keramischen Sintermassen auf den erhaltenen Apatit- und Wollastonit-Glaskeramiken titanoxidreiche Schichten auftreten, die die Eignung der erhaltenen Glaskeramiken als Knochenersatzmaterial erheblich einschränken. In diesem Zusammenhang werden TiO₂ -Anteile von 4 bis 5 Masse-% bereits als hoch, TiO₂ -Anteile von 7 Masse-% werden als sehr hoch eingeschätzt. Folglich werden TiO₂ -Anteile oberhalb 4 bis 5 Masse-%, zudem von 7 Masse-% und mehr üblicherweise vermieden, um die Ausscheidung titanoxidreicher Schichten an der Oberfläche eines gesinterten Formkörpers zu vermeiden.

Jedoch erwies sich, dass gemäß dem vorgeschlagenen Verfahren bei einem Zusatz an TiO₂ von 5 Masse-% oder mehr in das System zum Erhalt von Apatit- und Wollastonit-Glaskeramiken bisherige Nachteile des Auftretens vorgelagerter titanoxidreicher Schichten vermieden werden können. Überraschend erwies sich nämlich, dass unter Ausbleiben des Vorlagerns titanoxidreicher Schichten neben Apatit bzw. Wollastonit zusätzlich Titanit und Cristobalit in der gesinterten keramischen Masse vorliegen, wenn der keramischen Masse beispielsweise 7 Masse-% TiO₂ oder mehr, typischerweise bis zu 10 Masse-% zugesetzt werden. Im Ergebnis ist die Knochenkontaktrate so erhaltener Formkörper gegenüber jenen mit vorgelagerter titanoxidreicher Schicht erhöht und die Langzeitstabilität des Materials gleichzeitig gesteigert.

Unter den vorgeschlagenen Bedingungen werden diese vier Kristallphasen Apatit, Wollastonit, Titanit und Cristobalit synchron ausgeschieden, wobei die Vorlagerung einer TiO₂-reichen Schicht vorteilhafterweise unterbleibt. Das erhaltene keramische Material zeichnet sich durch eine hohe Bioaktivität aus. Unter Bioaktivität wird in diesem Zusammenhang die bindegewebsfreie knöcherne Fixation der betreffenden Keramik verstanden. Insbesondere wird darunter das Ein- oder Anwachsen von Knochen in oder an die Oberfläche der gesinterten keramischen Masse oder eines die vorgeschlagene keramische Masse umfassenden Knochenersatzmaterials bzw. eines die keramische Masse aufweisenden Implantats oder Formkörpers, bzw. einer mit diesen behandelten Oberfläche einer Endoprothese, das heißt die bindegewebsfreie Osseointegration im Organismus verstanden.

Dem Fachmann ist bekannt, dass durch unterschiedliche Verfahrenstechniken, insbesondere durch Wahl der Ausgangsgröße des Formkörpers bzw. seiner einzelnen Partikelbestandteile die Phasenzusammensetzung stark variiert werden kann, obwohl es sich um die gleiche stöchiometrische Zusammensatzung, ja sogar um die gleiche Ausgangscharge handeln kann. Dies soll in Tabelle 3 an folgendem Beispiel verdeutlicht werden. Ausgangsstoff ist die Zusammensetzung AWT7-o.K-Si.

**Tabelle 3**

| Ausgangsform | ca. Masse-% | | | | |
|---|---|---|---|---|---|
| | Apatit | Wollastonit | Titanit | Cristobalit | Glasphase |
| Glaskeramik (Partikelvolumen > 1cm³) 24h/720°C, 24h-920°C | 15 | 18 | 22 | 18 | 27 |
| Sinterglaskeramik Mahlgut 25-45µm 24h/720°C, 24h/920°C | 8 | 16 | 17 | 20 | 39 |
| Gepresster Formkörper Mahlgut d₅₀=1.4µm 24h/720°C, 24h/920°C | 12 | 21 | 23 | 14 | 30 |
| Gepresster Formkörper Mahlgut d₅₀=12,8µm 24h/720°C, 24h/920°C | 7 | 20 | 19 | 21 | 33 |
| 3D-Printing-Formkörper Mahlgut 45-100µm 24h/720°C, 24h/920°C | 5 | 17 | 21 | 23 | 33 |
| 3D-Printing-Formkörper Mahlgut 25-45µm 24h/720°C, 24h/920°C | 7 | 20 | 25 | 14 | 34 |
| Glaskeramik (Partikelvolumen > 1cm³) 14Tage/720°C, 13Tage/920°C | 14 | 14 | 20 | 19 | 34 |

Die Ursache für diese Variation liegt naturwissenschaftlich darin begründet, dass die Volumen zu Oberflächenkristallisation durch die gewählten Verfahrensbedingungen nachhaltig beeinflusst werden.

Die Ausscheidung der vier Hauptkristallphasen während des Sintervorgangs ist ebenso möglich, wenn als Bestandteile der Ausgangsform einzelne der Kristallite (Hauptkristallphasen), mehrere oder alle vier Hauptkristallphasen selbst in vermahlener Form verwendet werden. Unter Apatit wird dabei eine kristalline Verbindung gemäß der allgemeinen Formel Ca₅[(F, Cl, OH)|(PO₄)₃), unter Cristobalit - eine kristalline Verbindung der allgemeinen Formel SiO₂, unter Titanit - eine kristalline Verbindung gemäß der Formel CaTi[O|SiO₄] und unter Wollastonit - eine kristalline Verbindung der Formel CaSiO₃ bzw. Ca₃[Si₃O₉] verstanden. Es versteht sich von selbst, dass diese kristallinen Verbindungen Einlagerung von Spuren anderer Elemente, die bei der Kristallbildung aus den Ausgangsstoffen als begleitende Spurenelemente zugegen sind, aufweisen können. Die im verwendeten Mahlgut enthaltenen Ausgangsstoffe wiesen die Reinheitsstufe "p.a." auf und waren somit weitestgehend frei von Verunreinigungen.

Dem Fachmann ist auch bekannt, dass durch unterschiedliche Temperaturprofile beim Sintern die Phasenzusammensetzung stark variiert werden kann, obwohl es sich um die gleiche stöchiometrische Zusammensatzung, ja sogar um die gleiche Ausgangscharge handeln kann. Einen entsprechenden Beleg liefern die in Tab. 4 zusammengestellten Versuchsdaten.

**Tab. 4 Kristallphasen- und Glasanteile in ca. Masse%**

| AWT7-o.K-Si-Proben Sintervariationen | Apatit | Wollastonit | Titanit | Cristobalit | Glasphase |
|---|---|---|---|---|---|
| Glas | | | | | 100 |
| 950°C/1h | 12 | 12 | 20 | 14 | 42 |
| 1000°C/1h | 15 | 15 | 27 | 13 | 31 |
| 1050°C/1h | 16 | 16 | 31 | 15 | 22 |
| 1100°C/1h | 12 | 13 | 17 | 16 | 42 |
| 1150°C/1h | 12 | 16 | 23 | 24 | 24 |
| 1200°C/1h | 16 | 10 | 20 | 14 | 40 |

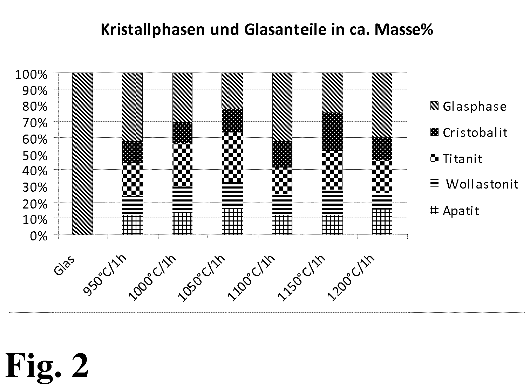

Eine grafische Darstellung der in Tab. 4 angeführten Werte ist in Fig. 2 gezeigt.

Dem Anwender wird somit ein langzeitstabileres glaskeramisches Material zur Verfügung gestellt, das entweder direkt in Form eines Granulates als Knochenersatz eingesetzt oder unter Anwendung verschiedener Prozesstechniken zu Formkörpern verarbeitet werden kann, die ebenfalls für den Knochenersatz relevante Eigenschaften besitzen. Ebenso kann das keramische Material für unterschiedliche Arten der Oberflächenbehandlung von Endoprothesen bzw. von Implantaten, insbesondere von metallischen Formkörpern verwendet werden.

Ein Beispiel für eine solche Oberflächenbehandlung ist das Bestrahlen nach Art des Sandstrahlens zum Aufrauhen einer Oberfläche der Endoprothese, wobei das dabei verwendete Strahlgut das vorgeschlagene keramische Material umfasst. Ein anderes Beispiel der Oberflächenbehandlung mit Hilfe der hier beschriebenen glaskeramischen Masse ist das Kugelstrahlen (Engl. - shot peening). Im Fall einer derartigen Anwendung umfasst das zum Kugelstrahlen eingesetzte Strahlmittel kompakte Formkörper des gesinterten keramischen Materials, vorzugsweise in Kugelform. Ein weiteres Beispiel einer Oberflächenbehandlung mit Hilfe der hier beschriebenen glaskeramischen Masse bzw. mit Formkörpern daraus ist das Polieren. Dabei wird die glaskeramische Masse oder daraus bestehende Schleifkörper als Poliermittel verwendet. Zum Polieren verwendete Schleifkörper können vorteilhafterweise eine Polyeder-Form, beispielsweise die Form von Prismen, Quadern oder eine Kugelform oder die Form von Ellipsoiden aufweisen. Das einsatzbereite Poliermittel kann beispielsweise Partikel von verschiedener Form und Größe umfassen, wobei die Form, die Größe und das Mischungsverhältnis verschiedener Partikel bzw. Schleifkörper durch die jeweiligen Charakteristika der zu polierenden Objekte bestimmt werden. Vorzugsweise werden die Schleifkörper zum Schleifen und/oder Polieren metallischer Teile von Endoprothesen verwendet.

Der gemeinsame Vorteil aller vorgeschlagenen Arten der Oberflächenbehandlung einer Endoprothese durch Kontakt mit einem Formkörper, umfassend eine keramische Masse der vorgeschlagenen Zusammensetzung, die zumindest zwei Sinterschritte durchlaufen hat, besteht darin, dass auf der Oberfläche der Endoprothese zurückbleibende Rückstände der keramischen Masse die Osseointegration der Endoprothese unterstützen, wohingegen üblicherweise als Strahlmittel eingesetzter Korund die biologische Kompatibilität der behandelten bzw. der mit dem Strahlmittel bestrahlten Oberfläche vermindert.

Die vorgeschlagene keramische Masse stellt eine neuartige Werkstoffkombination dar, die sich durch eine homogene Verteilung der kristallinen Phasen Apatit, Wollastonit, Titanit und Cristobalit in einer Glasphase auszeichnet.

Neben sich eröffnenden neuen wissenschaftlichen Fragestellungen ist dieser Werkstoff somit sowohl als Knochenersatz und Formkörper, als auch als stabiles und verbessertes Strahlgut für die Behandlung von Implantatoberflächen geeignet. Der gemäß dem Stand der Technik verwendete Sinterkorund weist den Nachteil auf, dass sich in bestrahlten Metallteilen, z.B. im Hüftgelenkendoprothesenstiel verankernde Korundpartikel, den direkten, bindegewebsfreien Knochenkontakt verhindern.

Es wurden keramische Massen in Form von Glaskeramiken unterschiedlicher Zusammensetzung und Eigenschaften hergestellt und untersucht, wobei die zum Erhalt der Glaskeramiken angesetzen Ausgansgsmischungen in der nachfolgenden Tabelle 5 zusammengefasst sind.

**Tab. 5 Stöchiometrische Zusammensetzung der AWT-Variationen (Angaben in Masse %)**

| Probe | SiO₂ | CaO | P₂O₅ | K₂O | Na₂O | MgO | CaF₂ | TiO₂ |
|---|---|---|---|---|---|---|---|---|
| AW [2] | 44,30 | 31,89 | 11,21 | 0,20 | 4,60 | 2,80 | 5,00 | - |
| AWT5 | 42,19 | 30,37 | 10,68 | 0,19 | 4,38 | 2,67 | 4,76 | 4,76 |
| AWT7 | 41,40 | 29,80 | 10,48 | 0,19 | 4,30 | 2,62 | 4,67 | 6,54 |
| AWT9 | 40,64 | 29,26 | 10,28 | 0,18 | 4,22 | 2,57 | 4,59 | 8,26 |
| AWT11 | 39,91 | 28,73 | 10,10 | 0,18 | 4,14 | 2,52 | 4,51 | 9,91 |
| AWT7-o.KNa | 43,17 | 31,07 | 10,94 | - | - | 3,41 | 4,87 | 6,54 |
| AWT7-o.K | 42,29 | 30,44 | 10,70 | - | 1,91 | 3,34 | 4,78 | 6,54 |
| AWT7-o.K-Si | 39,60 | 32,04 | 11,26 | - | 2,01 | 3,52 | 5,03 | 6,54 |

Als Ausgangsstoffe wurden SiO₂, CaCO₃, Ca₃(PO₄)₂, MgO, CaF₂, TiO₂; Na₂CO₃; K₂CO₃ eingewogen, eine Stunde im Taumelmischer homogenisiert und anschließend drei Stunden bei 1550 bis 1600°C geschmolzen. Die dünnflüssige Schmelze wurde je nach Weiterverarbeitung auf eine Stahlplatte gegossen oder gefrittet.

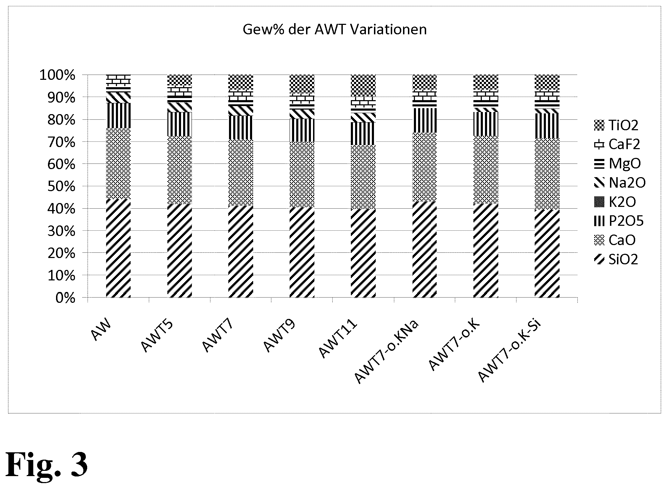

Dabei bedeutet das in der Tabelle verwendete Kürzel AW das nach dem Stand der Technik bekannte Apatit/Wollastonit [2] und AWT das gemäß der hiermit vorliegenden Beschreibung und der beispielhaft formulierten Ansprüche beim Sintern ausgebildete Apatit-Wollastonit-Titanit. Der dem Kürzel AWT nachfolgende Zahlenwert bezeichnet den Masseanteil weiterer zugesetzter Komponenten. Die Kürzel "AWT-o.K" bzw. "AWT-o.KNa" stehen dabei für Kalium-, bzw. Kalium- und Natriumfreie Mischungen. Die Werte aus Tab. 5 sind in Fig. 3 als Blockdiagramm wiedergegeben. Hier werden die Zusammensetzungsvariationen verdeutlicht.

Die vorgeschlagene Zusammensetzung stellt somit eine Kombination von kristallinen anorganischen Materialien bereit, die in geeigneter Weise die Anforderungen an einen langzeitstabilen Knochenersatz erfüllt. Insbesondere die geringe Löslichkeit, respektive die hohe chemische Beständigkeit des Materials unter physiologischen Bedingungen, eine ausgezeichnete Verarbeitbarkeit zu Formkörpern und die hohe Festigkeit dieser Formkörper, sowie das direkte, bindegewebsfreie Einwachsverhalten, d.h. die Bioaktivität der vorgeschlagenen keramischen Masse, stellen eine optimale Kombination von Merkmalen dar, die den Einsatz der keramischen Masse für die unterschiedlichsten Aspekte von Knochenersatzmaterialien empfiehlt. Dementsprechend wird vorgeschlagen, das keramische Material zu verwenden als Knochendefektfüllmaterial und zum Knochenaufbau (Augmentation), als Knochenersatz bzw. als Formkörper, als Strahlmittel und als Formkörper zum Aufrauhen und/oder Kugelstrahlen und/oder Polieren von Endoprothesen. Konkrete Ausführungsbeispiele betreffen die Behandlung der Oberfläche von Kieferimplantaten, Knie- und Hüftgelenksimplantaten, insbesondere die Aufrauhung eines Hüftgelenksendoprothesenstiels.

Zusammenfassend wird somit eine sinter- und/oder schmelzfähige keramische Masse, umfassend einen langzeitstabilen Verbund kristalliner Phasen von Apatit, Wollastonit, Titanit und optional Cristobalit vorgeschlagen, der durch eine Glasphase stabilisiert ist und ein Herstellungsverfahren dafür. Die keramische Masse ist durch Sintern eines Gemischs erhältlich, umfassend zumindest die Bestandteile SiO₂, CaO, P₂O₅, MgO, CaF₂ und TiO₂ allein oder in Kombination mit zumindest einem Alkalioxid, wobei das Alkalioxid ausgewählt ist unter Na₂O und K₂O. Selbstredend kann bei Bereitstellung einer Glasphase oder bei Bereitstellung von dem Fachmann bekannten Ausgangsstoffen für die Bildung einer Glasphase auch ein Ausgangsmaterial, umfassend die vermahlenen Kristallphasen Apatit, Wollastonit, Titanit und Cristobalt zur Herstellung der keramischen Masse verwendet werden. Weitere Ausführungsformen betreffen Verwendungen des gesinterten Materials in Form von Formkörpern zum Verfestigen, Reinigen, Aufrauen oder Polieren von Oberflächen medizinischer Implantate oder als Endoprothese.

Wenngleich hierin spezifische Ausführungsformen dargestellt und beschrieben worden sind, liegt es im Rahmen der vorliegenden Erfindung, die gezeigten Ausführungsformen geeignet zu modifizieren, ohne vom Schutzbereich der vorliegenden Erfindung abzuweichen. Die nachfolgenden Ansprüche stellen einen ersten, nicht bindenden Versuch dar, die Erfindung allgemein zu definieren.

### Referenzliste

[1] Kokubo, T. (1991): Bioactive Glass-Ceramics; Properties and Applications; Biomaterials, 12: 155-163.
[2] Berger, G.; Sauer, R.; Steinborn, G.; Wihsmann, F.G.; Thieme, V.; Köhler, St.; Dressel, H. (1989): Clinical application of surface reactive apatite/wollastonite containing glass-ceramics. in: XV. Int. Congress on Glass, Leningrad, USSR, 3.-7.7.1989, Volume 3a: 120-126.
[3] Berger, G.; Atzrodt, V. (1984): In vitro characterization of bioactivity of glasscrystalline implant materials using AUGER electron spectroscopy; physica status solidi (a), 85(1): 9-13.

## Patentansprüche

1. Keramische Masse, umfassend einen langzeitstabilen Verbund kristalliner Phasen von Apatit, Wollastonit, Cristobalit und Titanit, der durch eine Glasphase stabilisiert wird, wobei unter dem langzeitstabilen Verbund im Gegensatz zu einem resorbierbaren Material ein solches Material verstanden wird, das während eines Anwendungszeitraums, welcher einer typischen Lebensdauer eines Menschen entspricht, nicht resorbiert wird und nicht durch natürlichen Knochen ersetzt wird,
wobei zur Herstellung der keramischen Masse verwendete Ausgangsstoffe 35-45 Masse-% SiO₂, 25-35 Masse-% CaO, 10-15 Masse-% P₂O₅, 2-4 Masse-% MgO, 4-6 Masse-% CaF₂ und 5-10 Masse-% TiO₂ allein oder in Kombination mit zumindest einem Alkalioxid, ausgewählt unter 0,001-5 Masse-% Na₂O und 0,001-0,2 Masse-% K₂O umfassen,
wobei ein Anteil der Glasphase in der keramischen Masse nach einem Sintern weniger als 25 Masse-% beträgt, und
wobei die keramische Masse herstellbar ist durch
ein erstes Sintern drucklos bei einer Temperatur im Bereich von 650 °C bis 800 °C über eine Dauer von 1 bis 48 Stunden, ein Zermahlen der beim ersten Sintern gesinterten keramischen Masse, und ein zweites Sintern drucklos bei einer Temperatur im Bereich von 850 °C bis 1200 °C über eine Dauer von 1 bis 48 Stunden.

2. Keramische Masse nach Anspruch 1, wobei
das erste Sintern bei 720 °C über eine Dauer von 8 bis 24 Stunden erfolgt und das zweite Sintern bei 920 bis 1150 °C über eine Dauer von 18 bis 24 Stunden erfolgt.

3. Keramische Masse nach Anspruch 1 oder 2, wobei der Anteil der Glasphase in der keramischen Masse nach dem Sintern weniger als 10 Masse-% beträgt.

4. Herstellungsverfahren für eine keramische Masse, umfassend einen langzeitstabilen Verbund kristalliner Phasen von Apatit, Wollastonit, Cristobalit und Titanit, der durch eine Glasphase stabilisiert ist, umfassend:
- Bereitstellen einer Mischung, umfassend zumindest die Bestandteile 35-45 Masse-% SiO₂, 25-35 Masse-% CaO, 10-15 Masse-% P₂O₅, 2-4 Masse-% MgO, 4-6 Masse-% CaF₂ und 5-10 Masse-% TiO₂ allein oder in Kombination mit zumindest einem Alkalioxid, ausgewählt unter 0,001-0,2 Masse-% K₂O und 0,001-5 Masse-% Na₂O; wobei ein Masse-Verhältnis der Summe der Bestandteile SiO₂, CaO und P₂O₅ in der Mischung zur Masse des in der Mischung enthaltenen TiO₂ von 7:1 bis 20:1 beträgt;
- Vermahlen der Mischung;
- Sintern der vermahlenen Mischung, wobei das Sintern in einem ersten Sinterschritt drucklos bei einer Temperatur im Bereich von 650 °C bis 800 °C über eine Dauer von 1 bis 48 Stunden erfolgt;
- Zermahlen der gesinterten Mischung;
- Aufschlämmen der zermahlenen Mischung zu einem Schlicker;
- Formen eines Grünkörpers aus dem Schlicker;
- Sintern des Grünkörpers in einem zweiten Sinterschritt, wobei das Sintern des Grünkörpers drucklos bei einer Temperatur im Bereich von 850 bis 1200 °C über eine Dauer von 1 bis 48 Stunden erfolgt,
wobei unter dem langzeitstabilen Verbund im Gegensatz zu einem resorbierbaren Material ein solches Material verstanden wird, das während eines Anwendungszeitraums, welcher einer typischen Lebensdauer eines Menschen entspricht, nicht resorbiert wird und nicht durch natürlichen Knochen ersetzt wird, und
wobei ein Anteil der Glasphase in der keramischen Masse wenig er als 25 Masse-% beträgt.

5. Herstellungsverfahren nach Anspruch 4, wobei dem ersten Sinterschritt ein Schmelzen der vermahlenen Mischung bei einer Schmelztemperatur unterhalb von 1620 °C vorausgeht.

6. Herstellungsverfahren nach einem der Ansprüche 4 oder 5, wobei ein mittlerer Durchmesser von Partikeln der Bestandteile zum Erhalt der keramischen Masse 0,5 - 3000 µm, bevorzugt 0,5 - 3 µm; 2 - 10 µm; 10 - 100 µm; 50 - 1000 µm oder 300 - 3000 µm beträgt.

7. Herstellungsverfahren gemäß einem der Ansprüche 4 bis 6, weiterhin umfassend:
- Befüllen einer Sinterform mit der vermahlenen Mischung.

8. Herstellungsverfahren gemäß Anspruch 7, wobei das Sintern im ersten Sinterschritt bei einer Temperatur von 720 °C über eine Dauer von 8 bis 24 Stunden erfolgt, und im zweiten Schritt bei einer Temperatur von 920 bis 1150 °C über eine Dauer von 18 bis 24 Stunden erfolgt.

9. Herstellungsverfahren nach einem der Ansprüche 4 bis 8, wobei ein Masse-Verhältnis der Summe der Bestandteile SiO₂, CaO, P₂O₅, MgO und CaF₂ in der Mischung zur Masse des in der Mischung enthaltenen TiO₂ von 8:1 bis 20:1 beträgt.

10. Herstellungsverfahren nach einem der Ansprüche 4 bis 9, wobei das Formen des Grünkörpers schichtweise oder unter Verwendung eines additiven Verfahrens erfolgt.

11. Herstellungsverfahren nach zumindest einem der Ansprüche 4 bis 10, wobei das Zermahlen der ersten gesinterten Masse zuschlagsstofffrei erfolgt.

12. Verwendung einer keramischen Masse gemäß Anspruch 1 bis 3 als Feinstrahlmittel für medizinische Implantate, wobei eine mittlere Korngröße des Feinstrahlmittels im Bereich von 50 - 1000 µm liegt.

13. Verwendung einer keramischen Masse gemäß Anspruch 1 bis 3 als giessbare Masse in einem Schlickerguss-Verfahren, wobei eine mittlere Korngröße der in der giessbaren Masse dispergierten Teilchen im Bereich von
0,5 - 7 µm liegt.

14. Verwendung einer keramischen Masse gemäß Anspruch 1 bis 3 zum Verfestigen, Reinigen, Aufrauhen oder Polieren von Oberflächen medizinischer Implantate, wobei die keramische Masse als Formkörper vorliegt, und eine mittlere Ausdehnung des Formkörpers in einer Raumrichtung im Bereich von 50 µm bis 1 cm liegt, insbesondere im Bereich von 100 µm bis 2 mm liegt.

15. Verwendung einer keramischen Masse gemäß zumindest einem der Ansprüche 1 bis 3 als Schleifkörper für ein medizinisches Implantat, eine Endoprothese oder zur Behandlung einer metallischen Oberfläche.

16. Verwendung einer keramischen Masse gemäß Anspruch 1 bis 3 in einer gießbaren Masse für den Schlickerguss, wobei die keramische Masse diskrete Partikeln umfasst und ein mittlerer Durchmesser der Partikel 0,5 bis 3 µm umfasst.

17. Verwendung einer keramischen Masse gemäß Anspruch 1 bis 3 zur Herstellung eines Pressgranulats, wobei das Pressgranulat eine mittlere Granulatpartikelgröße von 0,5 µm bis 500 µm aufweist.

18. Verwendung einer keramischen Masse gemäß Anspruch 1 bis 3 zur Herstellung eines rieselfähigen Pulvers für ein additives Fertigungsverfahren , wobei das rieselfähige Pulver eine mittlere Pulverpartikelgröße von 10 µm bis 125 µm aufweist.

19. Verwendung einer keramischen Masse gemäß Anspruch 1 bis 3 als Strahlgut zur Oberflächenbehandlung, wobei die keramische Masse eine mittlere Partikelgröße von 50 µm bis 5000 µm aufweist.

20. Formkörper, umfassend eine keramische Masse gemäß zumindest einem der Ansprüche 1 bis 3, wobei der Formkörper ausgewählt ist unter: einem Mikropartikel, einer Kugel, einem Granulat, einem Polyeder, einer Scheibe, einem Diskus, einem Ellipsoid, einem Stab, einem Rohr, einem Zylinder, einem Kegel, einem knochenförmigen Gebilde oder einem Teil oder einem Abschnitt zumindest eines der genannten Formkörper.

## Claims

1. A ceramic mass, comprising a long-term stable compound of crystalline phases of apatite, wollastonite, cristobalite, and titanite, which is stabilized by a glass phase, wherein a long-term stabile compound is understood to be a material which, during an application period which corresponds to a typical life span of a human being, is not resorbed, and is not replaced by natural bone, in contrast to a resorbable material,
wherein the raw materials used for producing the ceramic mass comprise 35-45 mass% SiO₂, 25-35 mass% CaO, 10-15 mass% P₂O₅, 2-4 mass% MgO, 4-6 mass% CaF₂, and 5-10 mass% Ti02 alone or in combination with at least one alkali oxide selected from 0.001-5 mass% Na₂O and 0.001-0.2 mass% K₂O,
wherein a proportion of the glass phase in the ceramic mass after sintering is less than 25 mass%, and
wherein the ceramic mass is producible by
a first sintering step without pressure at a temperature in the range from 650°C to 800°C for a period of 1 to 48 hours, a grinding of the sintered ceramic mass obtained from the first sintering step, and a second sintering step without pressure at a temperature in the range from 850°C to 1200°C for a period of 1 to 48 hours.

2. The ceramic mass according to claim 1, wherein
the first sintering step is carried out at 720°C for a period of 8 to 24 hours, and the second sintering step is carried out at 920 to 1150°C for a period of 18 to 24 hours.

3. The ceramic mass according to claim 1 or 2, wherein the proportion of the glass phase in the ceramic mass after sintering is less than 10 mass%.

4. A production method for a ceramic mass, comprising a long-term stable compound of crystalline phases of apatite, wollastonite, cristobalite, and titanite, which is stabilized by a glass phase, comprising:
- providing a mixture comprising at least the components 35-45 mass% SiO₂, 25-35 mass% CaO, 10-15 mass% P₂O₅, 2-4 mass% MgO, 4-6 mass% CaF₂, and 5-10 mass% TiO₂ alone or in combination with at least one alkali oxide selected from 0.001-0.2 mass% K₂O and 0.001-5 mass% Na₂O, wherein a mass ratio of the sum of the components SiO₂, CaO, and P₂O₅ in the mixture to the mass of the Ti02 contained in the mixture is from 7:1 to 20:1;
- grinding the mixture;
- sintering the ground mixture, wherein the sintering is carried out in a first sintering step without pressure at a temperature in the range from 650°C to 800°C for a period of 1 to 48 hours;
- pulverizing the sintered mixture;
- slurrying the pulverized mixture into a slurry;
- forming a green body from the slurry;
- sintering the green body in a second sintering step, wherein the sintering of the green body is carried out without pressure at a temperature in the range from 850 to 1200°C for a period of 1 to 48 hours;
wherein a long-term stabile compound is understood to be a material which, during an application period which corresponds to a typical life span of a human being, is not resorbed, and is not replaced by natural bone, in contrast to a resorbable material, and wherein a proportion of the glass phase in the ceramic mass is less than 25 mass%.

5. The production method according to claim 4, wherein a melting of the ground mixture at a melting temperature below 1620°C precedes the first sintering step.

6. The production method according to claim 4 or 5, wherein an average diameter of particles of the components for obtaining the ceramic mass is 0.5 - 3000 µm, preferably 0.5 - 3 µm; 2 - 10 µm; 10 - 100 µm; 50 - 1000 µm; or 300 - 3000 µm.

7. The production method according to one of claims 4 to 6, additionally comprising:
- filling a sintering mold with the ground mixture.

8. The production method according to claim 7, wherein the sintering in the first sintering step is carried out at a temperature of 720°C for a period of 8 to 24 hours, and in the second sintering step at a temperature from 920 to 1150°C for a period of 18 to 24 hours.

9. The production method according to one of claims 4 to 8, wherein a mass ratio of the sum of the components SiO₂, CaO, P₂O₅, MgO, and CaF₂ in the mixture to the mass of the TiO₂ contained in the mixture is from 8:1 to 20:1.

10. The production method according to one of claims 4 to 9, wherein the shaping of the green body is carried out in layers or by using an additive method.

11. The production method according to one of claims 4 to 10, wherein the pulverization of the first sintered mass is carried out free of additives.

12. A use of a ceramic mass according to claims 1 to 3 as fine blasting means for medical implants, wherein an average grain size of the fine blasting means lies in the range of 50 - 1000 µm.

13. A use of a ceramic mass according to claims 1 to 3 as a castable composition in a slip casting method, wherein an average grain size of the particles dispersed in the castable composition lies in the range from 0.5 - 7 µm.

14. A use of a ceramic mass according to claims 1 to 3 for hardening, cleaning, roughening, or polishing surfaces of medical implants, wherein the ceramic mass is present as a shaped body, and an average extension of the shaped body in one spatial direction lies in the range from 50 µm to 1 cm, in particular lies in the range from 100 µm to 2 mm.

15. A use of a ceramic mass according to at least one of claims 1 to 3 as a grinding body for a medical implant, an endoprosthesis, or for treating a metallic surface.

16. A use of a ceramic mass according to claims 1 to 3 in a castable composition for slip casting, wherein the ceramic mass comprises discrete particles and an average diameter of the particles comprises 0.5 to 3 µm.

17. A use of a ceramic mass according to claims 1 to 3 for producing a pressing granulate, wherein the pressing granulate has an average granulate particle size of 0.5 µm to 500 µm.

18. A use of a ceramic mass according to claims 1 to 3 for producing a free-flowing powder for an additive manufacturing method, wherein the free-flowing powder has an average powder particle size of 10 µm to 125 µm.

19. A use of a ceramic mass according to claims 1 to 3 as blasting material for surface treatment, wherein the ceramic mass has an average particle size of 50 µm to 5000 µm.

20. A shaped body comprising a ceramic mass according to at least one of claims 1 to 3, wherein the shaped body is selected from: a microparticle, a sphere, a granule, a polyhedron, a wafer, a disk, an ellipsoid, a rod, a tube, a cylinder, a cone, a bone-shaped structure, or a part or a section of at least one of the listed shaped bodies.

## Revendications

1. Masse céramique comprenant un composite stable à long terme de phases cristallines d'apatite, de wollastonite, de cristobalite et de titanite, qui est stabilisé par une phase vitreuse, dans laquelle on entend par composite stable à long terme, au contraire d'un matériau résorbable, un matériau qui, au cours d'une durée d'utilisation correspondant à une durée de vie typique d'un être humain, n'est pas résorbé et n'est pas remplacé par de l'os naturel,
les matières de départ utilisées pour la fabrication de la masse céramique comprenant 35 à 45 % en masse de SiO₂, 25 à 35 % en masse de CaO, 10 à 15 % en masse de P₂O₅, 2 à 4 % en masse de MgO, 4 à 6 % en masse de CaF₂ et 5 à 10 % en masse de TiO₂, seul ou en combinaison avec au moins un oxyde de métal alcalin choisi parmi 0,001 à 5 % en masse de Na₂O et 0,001 à 0,2 % en masse de K₂O,
une proportion de la phase vitreuse dans la masse céramique étant, après un frittage, inférieure à 25 % en masse, et
la masse céramique pouvant être fabriquée par un premier frittage sans pression à une température dans la plage de 650 °C à 800 °C sur une durée de 1 à 48 heures, un broyage de la masse céramique frittée lors du premier frittage, et un second frittage sans pression à une température dans la plage de 850 °C à 1200 °C sur une durée de 1 à 48 heures.

2. Masse céramique selon la revendication 1, le premier frittage étant réalisé à 720 °C sur une durée de 8 à 24 heures, et le second frittage étant réalisé à 920 à 1150 °C sur une durée de 18 à 24 heures.

3. Masse céramique selon la revendication 1 ou 2, la proportion de la phase vitreuse dans la masse céramique après le frittage étant inférieure à 10 % en masse.

4. Procédé de fabrication d'une masse céramique comprenant un composite stable à long terme de phases cristallines d'apatite, de wollastonite, de cristobalite et de titanite, qui est stabilisé par une phase vitreuse, comprenant :
- la fourniture d'un mélange comprenant au moins les constituants 35 à 45 % en masse de SiO₂, 25 à 35 % en masse de CaO, 10 à 15 % en masse de P₂O₅, 2 à 4 % en masse de MgO, 4 à 6 % en masse de CaF₂ et 5 à 10 % en masse de TiO₂, seul ou en combinaison avec au moins un oxyde de métal alcalin choisi parmi 0,001 à 0,2 % en masse de K₂O et 0,001 à 5 % en masse de Na₂O, un rapport en masse de la somme des constituants SiO₂, CaO et de P₂O₅ dans le mélange à la masse du TiO₂ contenu dans le mélange étant de 7:1 à 20:1 ;
- le broyage du mélange ;
- le frittage du mélange broyé, le frittage étant réalisé dans une première étape de frittage sans pression à une température dans la plage de 650 °C à 800 °C sur une durée de 1 à 48 heures ;
- le broyage du mélange fritté ;
- la mise en suspension du mélange broyé pour former une barbotine ;
- la mise en forme d'une ébauche crue à partir de la barbotine,
- le frittage de l'ébauche crue dans une seconde étape de frittage, le frittage de l'ébauche crue étant réalisée par frittage sans pression à une température dans la plage de 850 à 1200 °C sur une durée de 1 à 48 heures,
dans laquelle on entend par composite stable à long terme, au contraire d'un matériau résorbable, un matériau qui, au cours d'une durée d'utilisation correspondant à une durée de vie typique d'un être humain, n'est pas résorbé et n'est pas remplacé par de l'os naturel, et
une proportion de la phase vitreuse dans la masse céramique étant inférieure à 25 % en masse.

5. Procédé de fabrication selon la revendication 4, dans lequel la première étape de frittage est précédée d'une fusion du mélange broyé, à une température de fusion inférieure à 1620 °C.

6. Procédé de fabrication selon l'une des revendications 4 ou 5, dans lequel un diamètre moyen des particules des constituants pour obtenir la masse céramique est de 0,5 à 3000 µm, de préférence de 0,5 à 3 µm ; 2 à 10 µm ; 10 à 100 µm ; 50 à 1000 µm ou 300 à 3000 µm.

7. Procédé de fabrication selon l'une des revendications 4 à 6, comprenant en outre :
- le remplissage d'un moule de frittage avec le mélange broyé.

8. Procédé de fabrication selon la revendication 7, dans lequel le frittage est réalisé dans la première étape de frittage à une température de 720 °C sur une durée de 8 à 24 heures, et dans la deuxième étape est réalisé à une température de 920 à 1150 °C sur une durée de 18 à 24 heures.

9. Procédé de fabrication selon l'une des revendications 4 à 8, dans lequel un rapport en masse de la somme des constituants SiO₂, CaO et de P₂O₅ dans le mélange à la masse du TiO₂ contenu dans le mélange est de 8:1 à 20:1.

10. Procédé de fabrication selon l'une des revendications 4 à 9, dans lequel la mise en forme de l'ébauche crue est réalisée couche par couche ou par utilisation d'un procédé additif.

11. Procédé de fabrication selon au moins l'une des revendications 4 à 10, dans lequel le broyage de la première masse frittée est réalisé sans additifs.

12. Utilisation d'une masse céramique selon les revendications 1 à 3 en tant qu'agent de sablage fin pour implants médicaux, une granulométrie moyenne de l'agent de sablage fin étant comprise dans la plage de 50 à 1000 µm.

13. Utilisation d'une masse céramique selon les revendications 1 à 3 en tant que masse coulable dans un procédé de coulage en barbotine, une granulométrie moyenne des particules dispersées dans la masse coulable étant comprise dans la plage de 0,5 à 7 µm.

14. Utilisation d'une masse céramique selon les revendications 1 à 3 pour consolider, nettoyer, rendre rugueuses ou polir des surfaces d'implants médicaux, la masse céramique se présentant sous forme d'un corps formé, et une extension moyenne du corps formé dans une direction de l'espace étant comprise dans la plage de 50 µm à 1 cm, en particulier dans la plage de 100 µm à 2 mm.

15. Utilisation d'une masse céramique selon au moins l'une des revendications 1 à 3 en tant que corps abrasif aggloméré pour un implant médical, une endoprothèse, ou pour le traitement d'une surface métallique.

16. Utilisation d'une masse céramique selon les revendications 1 à 3 dans une masse coulable pour le coulage en barbotine, la masse céramique comportant des particules discrètes et présentant un diamètre moyen des particules de 0,5 à 3 µm.

17. Utilisation d'une masse céramique selon les revendications 1 à 3 pour la fabrication d'un granulé pressé, le granulé pressé présentant une granulométrie moyenne des particules de 0,5 µm à 500 µm.

18. Utilisation d'une masse céramique selon les revendications 1 à 3 pour la fabrication d'une poudre à écoulement libre pour un procédé de fabrication additive, la poudre à écoulement libre ayant une taille moyenne de particules de poudre de 10 µm à 125 µm.

19. Utilisation d'une masse céramique selon les revendications 1 à 3 en tant que matériau de sablage pour le traitement de surface, la masse céramique ayant une taille moyenne de particules de 50 µm à 5000 µm.

20. Corps formé comprenant une masse céramique selon au moins l'une des revendications 1 à 3, le corps formé étant choisi parmi une microparticule, une sphère, un granule, un polyèdre, une plaque, un disque, un ellipsoïde, une barre, un tube, un cylindre, un cône, une structure en forme d'os ou une partie ou portion d'au moins l'un des corps formés mentionnés.
